# EUROPÄISCHE PATENTSCHRIFT

(11) **EP 4 282 243 B1**
(45) Veröffentlichungstag und Bekanntmachung des Hinweises auf die Patenterteilung: **28.01.2026**
(21) Anmeldenummer: 23171619.2
(22) Anmeldetag: 04.05.2023
(51) Int. Cl.: A01B 79/00, B62D 55/065

(54) **VERFAHREN ZUR ERMITTLUNG EINER BODENBESCHAFFENHEIT**
METHOD FOR DETERMINING THE CONDITION OF A SOIL
PROCÉDÉ DE DÉTERMINATION D'UNE CONDITION DE SOL

(30) Priorität: 27.05.2022 DE 102022113393
(43) Veröffentlichungstag der Anmeldung: 29.11.2023
(73) Patentinhaber: Deere & Company, Moline, IL 61265 (US)
(72) Erfinder: BROCKE, Stefan, 68163 Mannheim (DE); SCHOTT, Florian, 68163 Mannheim (DE)
(74) Vertreter: Dehnhardt, Florian Christopher

(56) Entgegenhaltungen:
- US-A1- 2018 265 145
- US-A1- 2021 173 399

## Beschreibung

Die Erfindung betrifft ein Verfahren zur Ermittlung einer Bodenbeschaffenheit.

Das Wissen um die Bodenbeschaffenheit einer zu bearbeitenden Ackeroberfläche ist wesentlich für die korrekte Anwendung landwirtschaftlicher Bearbeitungsmethoden bzw. -techniken. Dies schlägt sich unter anderem in der Auswahl geeigneter Bodenbearbeitungsgeräte bzw. Arbeitsparameter, wie beispielsweise der Festlegung der Bearbeitungstiefe bodeneingreifender Arbeitswerkzeuge an einem Pflug, einem Grubber, einer Sämaschine oder dergleichen, ebenso nieder wie hinsichtlich der Frage eines zu wählenden Reifenfülldrucks, eines optimalen Aussaat- bzw. Erntezeitpunkts, oder der effizienten und umweltschonenden Unkrautbekämpfung.

Die Bodenbeschaffenheit wird hierbei unter anderem durch die Bodenstruktur, Bodenorganismen (biologische Aktivität), den Feuchtegehalt sowie den Verdichtungsgrad der Bodenoberfläche geprägt. Im Normalfall erfolgt eine Abschätzung visuell durch den landwirtschaftlichen Anwender, was entsprechende Erfahrung voraussetzt, oder aber auf Grundlage stichprobenartig durchgeführter Bodenmessungen mittels stationärer oder mobiler Analyseeinrichtungen.

Angesichts dessen ist es Aufgabe der vorliegenden Erfindung, ein Verfahren der eingangs genannten Art dahingehend anzugeben, dass eine kontinuierliche Bereitstellung von die Bodenbeschaffenheit einer zu bearbeitenden Ackeroberfläche betref-Ein Verfahren zur Ermittlung einer Bodenbeschaffenheit nach dem Oberbegriff des Anspruchs 1 ist aus der Druckschrift US 2021/173399 A1 bekannt.

Diese Aufgabe wird durch ein Verfahren zur Ermittlung einer Bodenbeschaffenheit mit den Merkmalen des Patentanspruchs 1 gelöst. Vorteilhafte Weiterbildungen des erfindungsgemäßen Verfahrens gehen aus den Unteransprüchen hervor.

Das erfindungsgemäße Verfahren zur Ermittlung einer Bodenbeschaffenheit sieht vor, dass mittels einer Erfassungseinrichtung ein durch Einsinken verursachtes kinematisches Verhalten eines Raupenlaufwerks beim Befahren eines kompressiblen Untergrunds beobachtet wird, wobei von einer Bewertungseinrichtung ausgehend von dem beobachteten kinematischen Verhalten des Raupenlaufwerks auf wenigstens einen die Bodenbeschaffenheit charakterisierenden Parameter geschlossen wird.

Diese Vorgehensweise macht sich den Umstand zunutze, dass mit steigenden Anforderungen an die Schonung des Ackerbodens land- und forstwirtschaftliche Arbeitsmaschinen bzw. Arbeitsgeräte zunehmend mit Raupenlaufwerken ausgestattet werden. Diese umfassen typischerweise einen aus faserverstärktem Gummi hergestellten Laufwerkriemen, der an einem Fahrgestell über eine Vielzahl angetriebener bzw. frei rotierender Umlenk-, Spann- und Stützrollen geführt ist. Je nach Komplexität des Fahrgestells ist zumindest ein Teil der Rollen derart gelenkig bzw. gefedert aufgehängt, dass der Verlauf des Laufwerkriemens sich den Unebenheiten des befahrenen Untergrunds anpassen kann. Hierbei hat die Art der Anlenkung bzw. Federung der Rollen signifikanten Einfluss auf die Druckverteilung zwischen Laufwerkriemen und Untergrund, wobei diese unter der Wirkung der am Laufwerkriemen auftretenden Antriebsmomente meist asymmetrisch ist. Insofern weist jeder Raupenlaufwerkstyp bzw. jedes Raupenlaufwerksmodell eine spezifische bzw. charakteristische Signatur hinsichtlich seines Kompressionseffekts auf den jeweils befahrenen Untergrund auf. Auf diese Weise ist es möglich, während des Fahrtbetriebs einer mit einem Raupenlaufwerk ausgestatteten Arbeitsmaschine bzw. eines damit ausgestatteten Arbeitsgeräts durch Erfassung des beim Einsinken in den Untergrund beobachteten kinematischen Verhaltens des Raupenlaufwerks kontinuierlich auf die Bodenbeschaffenheit bzw. die hierfür repräsentativen Parameter zu schließen. Hierzu wird das mittels der Erfassungseinrichtung beobachtete kinematische Verhalten einem für das betreffende Raupenlaufwerk vorgegebenen Vorhersagemodell zugeführt, anhand dessen wiederum eine Aussage hinsichtlich der Bodenbeschaffenheit getroffen wird.

Da der von dem jeweiligen Raupenlaufwerk verursachte Kompressionseffekt ebenfalls abhängig ist vom Umfang des am Laufwerkriemen gegenüber dem Untergrund antriebsbedingt auftretenden Traktionsmoments und/oder der in einem Anlenkpunkt des Raupenlaufwerks gegenüber einem Fahrzeugchassis gewichtsbedingt wirkenden Achslast, können zur Verbesserung der Genauigkeit des Vorhersagemodells weiterhin entsprechende Informationen eines Antriebssystems und/oder eines Achslastsensors Berücksichtigung finden, um den vorgenannten Einflüssen Rechnung tragen.

Das Vorhersagemodell wird vorab aufgrund empirisch durchgeführter Test- und Messreihen eingelernt und in eine zur Ausführung des erfindungsgemäßen Verfahrens vorgesehene Prozessoreinheit, die Bestandteil der Bewertungseinrichtung ist, hochgeladen. Das Vorhersagemodell stellt eine eindeutige Korrelation zwischen dem beobachteten kinematischen Verhalten des Raupenlaufwerks und der jeweiligen Bodenbeschaffenheit her. Zur Verbesserung des Vorhersagehorizonts bietet sich zudem der Einsatz entsprechender KI-Verfahren (KI - Künstliche Intelligenz) an.

Bei der Arbeitsmaschine kann es sich um einen landwirtschaftlichen Traktor, eine Erntemaschine oder ein forstwirtschaftliches Arbeitsfahrzeug handeln. Daneben ist der Einsatz des erfindungsgemäßen Verfahrens auch bei land- oder forstwirtschaftlichen Arbeitsgeräten, wie angetriebenen Ladeanhängern oder dergleichen, denkbar.

Das kinematische Verhalten ergibt sich aufgrund einer Auslenkung, die fahrtbedingt an einer gegenüber dem Untergrund beweglich gelagerten Fahrgestellkomponente des Raupenlaufwerks auftritt. Erfindungsgemäß ist das Fahrgestell mittels eines Basisteils über ein Drehgelenk schwenkbar an einem Fahrzeugchassis angebracht, sodass sich die zu beobachtende Auslenkung aus einer mittels der Erfassungseinrichtung sensorisch erfassbaren Winkeländerung um die Drehgelenkachse ergibt. Ein am Laufwerkriemen antriebsbedingt gegenüber dem Untergrund auftretendes Traktionsmoment bewirkt ein asymmetrisches Einsinken des Raupenlaufwerks entgegen der Fahrtrichtung, was sich in einer entsprechenden Winkeländerung äußert. Wie bereits erwähnt, können bei komplexen Laufwerksanordnungen auch weitere bewegliche Fahrgestellkomponenten vorhanden sein, die der Federung und/oder dem Spannen des Laufwerkriemens mittels zugehöriger Umlenk-, Spann- und Stützrollen dienen. Aus der umfassenden sensorischen Erfassung der insoweit auftretenden Bewegungen ergibt sich ein komplexes und damit besonders unterscheidungskräftiges Auslenkungsmuster, das einen genauen Rückschluss auf die jeweilige Bodenbeschaffenheit zulässt.

Des Weiteren ist es möglich, dass sich das kinematische Verhalten aufgrund einer Normal- und/oder Tangentialbeschleunigung, die fahrtbedingt an einem dem Untergrund zugewandten Abschnitt des Laufwerkriemens auftritt, ergibt. Die Kenntnis der Normal- und/oder Tangentialbeschleunigung kann von der Bewertungseinrichtung zur Ermittlung der Orientierung des betreffenden Riemenabschnitts gegenüber dem Untergrund dienen. Diese Vorgehensweise ist weitgehend unabhängig von der konkreten baulichen Ausgestaltung des Fahrgestells und kann redundant zur auslenkungsbasierten Beurteilung des kinematischen Verhaltens des Raupenlaufwerks herangezogen werden.

Die Normal- und/oder Tangentialbeschleunigung kann mittels eines in den Riemenabschnitt eingebetteten Beschleunigungssensors als Bestandteil der Erfassungseinrichtung bestimmt werden, wobei ein diese repräsentierender Datenstrom drahtlos an die Bewertungseinrichtung übermittelt wird. Bevorzugt ist der Beschleunigungssensor vor mechanischen Einflüssen geschützt auf einer Innenseite des Laufwerkriemens untergebracht bzw. darin eingebettet. Hierbei kann der Beschleunigungssensor sowie eine zur Datenübertragung vorgesehene Funkschnittstelle aus einem eingebauten Akkumulator, der sich induktiv und damit kontaktlos von außen aufladen lässt, oder einem unter der Bewegung des Laufwerkriemens stromerzeugenden Generator mit elektrischer Energie versorgt werden. Beschleunigungssensor, Funkschnittstelle und Akkumulator bzw. stromerzeugender Generator sind beispielsweise in ein gemeinsames Beschleunigungsmessmodul integriert.

Als Bezugssystem zur Ermittlung der Orientierung des Riemenabschnitts dient typischerweise das Fahrzeugchassis. Hierzu ist diesem ein eigener Beschleunigungssensor zur Bestimmung einer Referenz-Orientierung zugeordnet.

Da der Laufwerkriemen entlang seines Verlaufs mehrfach umgelenkt wird, ist es zur unterbrechungsfreien Erfassung der Normal- und/oder Tangentialbeschleunigung denkbar, eine den Umlenkstellen entsprechende Anzahl an Beschleunigungssensoren entlang des Laufwerkriemens verteilt anzuordnen.

Im Hinblick auf eine möglichst genaue Ermittlung der Bodenbeschaffenheit kann vorgesehen sein, dass das beobachtete kinematische Verhalten des Raupenlaufwerks von der Bewertungseinrichtung hinsichtlich von in Fahrtrichtung liegenden Bodenunebenheiten bereinigt wird. Die Erfassung der Bodenunebenheiten kann mittels vorausschauender Sensoreinrichtungen zur dreidimensionalen Umgebungserfassung, beispielsweise in Gestalt einer Stereokamera oder eines Lidars, erfolgen.

Typischerweise handelt es sich bei den die Bodenbeschaffenheit charakterisierenden Parametern um Informationen hinsichtlich einer Bodenfeuchtigkeit, einer Bodenstruktur, einer Bodenverdichtung und/oder eines Bodentyps. Hierbei kann die Ermittlung der Bodenbeschaffenheit auch auf eine Auswahl der vorstehend aufgeführten Parameter beschränkt sein, wobei insbesondere Angaben hinsichtlich eines aktuellen Feuchtegehalts, mithin der Bodenfeuchtigkeit, aus alternativen Datenquellen stammen und so zu einer verbesserten Genauigkeit der verbleibenden Parameter beitragen können. Der Feuchtegehalt ergibt sich beispielsweise aus Wetterinformationen oder einer vor Ort durchgeführten Bodenmessung.

Um die im Hinblick auf die Bodenbeschaffenheit gewonnenen Informationen insbesondere beim erneuten Befahren der selben Ackerfläche nutzbar zu machen, ist es möglich, dass die ermittelten Parameter mittels eines mit der Bewertungseinrichtung kommunizierenden Navigationssystems durch Verknüpfung mit einer zugehörigen kartografischen Position verortet und als Datensatz an einen zentralen Datenserver übermittelt werden. Von dort können diese zu Zwecken der Schlagplanerstellung bzw. Prozessoptimierung von einem Anwender heruntergeladen werden.

Das erfindungsgemäße Verfahren zur Ermittlung einer Bodenbeschaffenheit wird im Folgenden anhand der Zeichnungen näher beschrieben. Dabei beziehen sich identische Bezugszeichen auf übereinstimmende oder bezüglich ihrer Funktion vergleichbare Komponenten. Es zeigen:
- Fig. 1: eine in einem landwirtschaftlichen Traktor befindliche Anordnung zur Ausführung des erfindungsgemäßen Verfahrens zur Ermittlung einer Bodenbeschaffenheit, und
- Fig. 2: ein als Blockdiagramm veranschaulichtes Ausführungsbeispiel des erfindungsgemäßen Verfahrens.

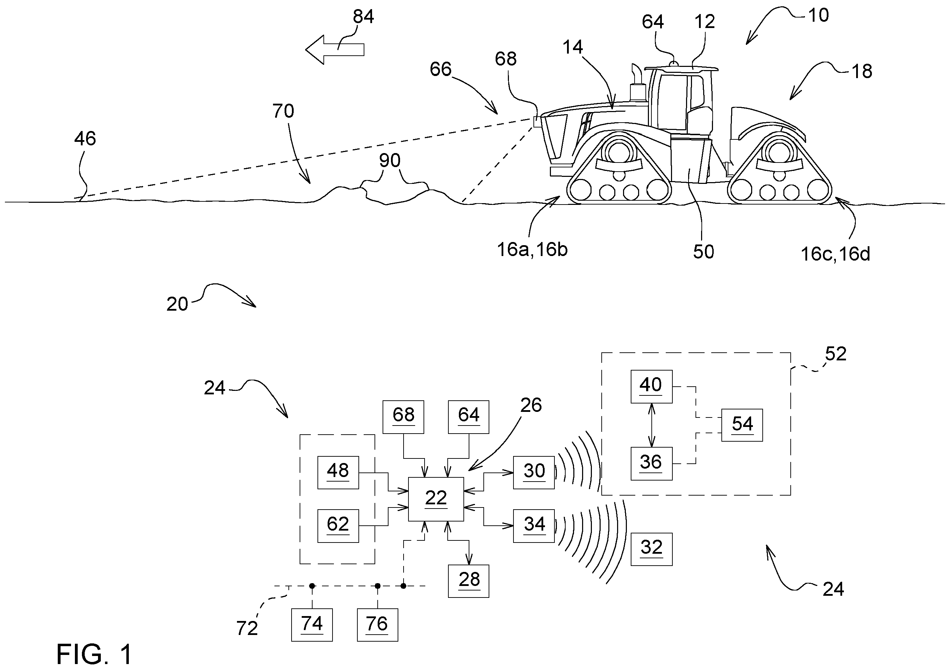

Fig. 1 zeigt eine in einem landwirtschaftlichen Traktor untergebrachte Anordnung zur Ausführung des erfindungsgemäßen Verfahrens zur Ermittlung einer Bodenbeschaffenheit.

Bei dem landwirtschaftlichen Traktor 10 handelt es sich vorliegend um einen knickgelenkten Vier-Raupentraktor 12, bei dem einer Vorderachse 14 ein erstes Paar angetriebener Raupenlaufwerke 16a, 16b und einer Hinterachse 18 ein zweites Paar angetriebener Raupenlaufwerke 16c, 16d zugeordnet ist. Hierbei sind aufgrund der gewählten Ansicht lediglich die auf der linken Seite des landwirtschaftlichen Traktors 10 vorgesehenen Raupenlaufwerke 16a, 16c zu sehen, die der rechten Seite zugeordneten Raupenlaufwerke 16b, 16d sind entlang der Sichtlinie verdeckt und zu den beiden Raupenlaufwerken 16a, 16c spiegelbildlich angeordnet.

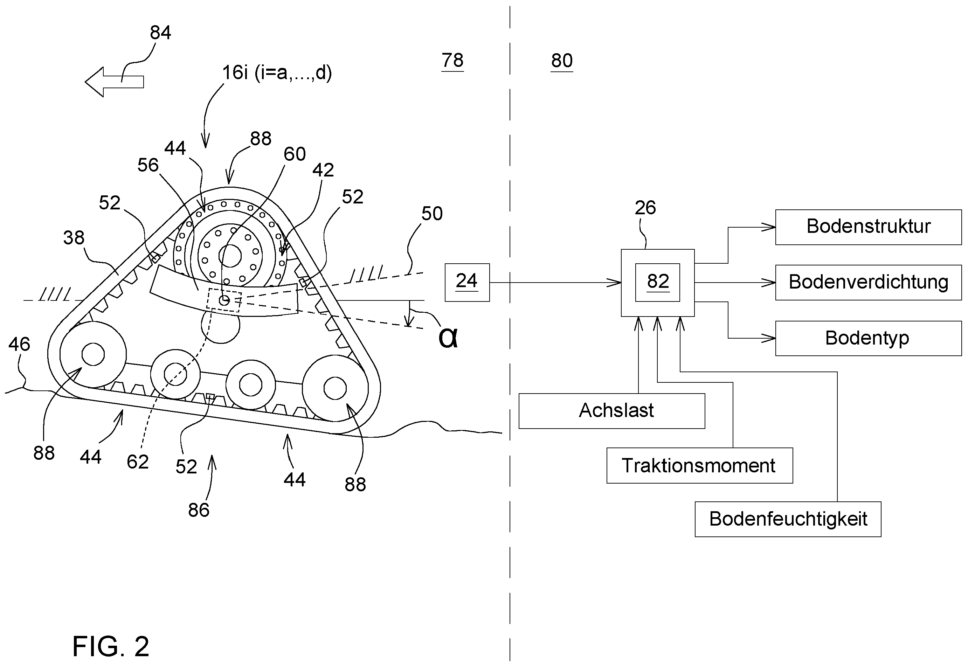

Die Anordnung 20 umfasst eine Prozessoreinheit 22, die Bestandteil einer mit einer Erfassungseinrichtung 24 kommunizierenden Bewertungseinrichtung 26 ist und mit einer Speichereinheit 28 sowie über eine erste Drahtlosschnittstelle 30 mit einem zentralen Datenserver 32 bzw. über eine zweite Drahtlosschnittstelle 34 mit einer Funkschnittstelle 36 eines in einen aus faserverstärktem Gummi hergestellten Laufwerkriemen 38 eingebetteten ersten Beschleunigungssensors 40 (siehe Fig. 2) in Datenaustauschverbindung steht.

Entsprechend der Darstellung eines einzelnen Raupenlaufwerks 16i (i = a,...,d) in Fig. 2, ist der Laufwerkriemen 38 an einem Fahrgestell 42 über eine Vielzahl angetriebener bzw. frei rotierender Umlenk-, Spann- und Stützrollen 44 geführt. Je nach Komplexität des Fahrgestells 42 ist zumindest ein Teil der Rollen 44 derart gelenkig bzw. gefedert aufgehängt, dass der Verlauf des Laufwerkriemens 38 sich den Unebenheiten eines befahrenen Untergrunds 46 anpassen kann.

Daneben ist ein mit der Prozessoreinheit 22 verbundener zweiter Beschleunigungssensor 48 vorhanden. Dieser ist an einem Fahrzeugchassis 50 des landwirtschaftlichen Traktors 10 angebracht und gemeinsam mit dem ersten Beschleunigungssensor 40 Bestandteil der Erfassungseinrichtung 24.

Der erste Beschleunigungssensor 40 sowie die Funkschnittstelle 36 sind in ein gemeinsames Beschleunigungsmessmodul 52 integriert und werden mittels eines eingebauten Akkumulators 54, der sich induktiv und damit kontaktlos von außen aufladen lässt, mit elektrischer Energie versorgt. Hierbei ist das Beschleunigungsmessmodul 52 vor mechanischen Einflüssen geschützt auf einer Innenseite des Laufwerkriemens 38 eingebettet.

Wie am besten in Fig. 2 zu erkennen ist, ist das Fahrgestell 42 des Raupenlaufwerks 16i mittels eines Basisteils 56 über ein Drehgelenk 58 schwenkbar an dem Fahrzeugchassis 50 angebracht, wobei eine entlang einer Drehgelenkachse 60 auftretende Winkeländerung α mittels eines inkrementalen Drehgebers 62, der ebenfalls von der Erfassungseinrichtung 24 umfasst ist, sensorisch erfasst wird.

Des Weiteren dient ein GPS-basiertes Navigationssystem 64 der Ermittlung einer aktuellen kartografischen Position des landwirtschaftlichen Traktors 10, wobei die jeweils ermittelte kartografische Position in Form entsprechender Positionsinformationen an die Prozessoreinheit 22 übertragen wird.

Beispielsgemäß sind im Frontbereich 66 des landwirtschaftlichen Traktors 10 vorausschauende Sensoreinrichtungen 68 angebracht. Diese sind in Gestalt einer Stereokamera oder eines Lidars ausgebildet und dienen der Voraberfassung einer Oberflächenkontur 70 des zu befahrenden Untergrunds 46. Die erfasste Oberflächenkontur 70 wird der Prozessoreinheit 22 in Form entsprechender Konturinformationen zur Verfügung gestellt.

Daneben hat die Prozessoreinheit 22 über einen CAN-Datenbus 72 Zugriff auf Informationen eines Antriebssystems 74 und/oder eines Achslastsensors 76 des landwirtschaftlichen Traktors 10.

In Fig. 2 ist ein als Blockdiagramm veranschaulichtes Ausführungsbeispiel des erfindungsgemäßen Verfahrens wiedergegeben, das nachfolgend unter Bezugnahme auf Fig. 1 erläutert werden soll. Hierbei ist stellvertretend ein einzelnes Raupenlaufwerk 16i (i = a,...,d) dargestellt. Um die Qualität der Ermittlung der Bodenbeschaffenheit zu verbessern, wird das erfindungsgemäße Verfahren allerdings bevorzugt unter Einbeziehung sämtlicher vorhandener Raupenlaufwerke 16a,...,16d des landwirtschaftlichen Traktors 10 durchgeführt.

Der Ablauf des Verfahrens lässt sich im Wesentlichen in zwei Funktionsblöcke gliedern. So wird in einem ersten Funktionsblock 78 mittels der Erfassungseinrichtung 24 beim Befahren des als kompressibel angenommenen Untergrunds 46 ein durch Einsinken verursachtes kinematisches Verhalten des Raupenlaufwerks 16i beobachtet, woraufhin in einem zweiten Funktionsblock 80 von der Bewertungseinrichtung 26 bzw. der von dieser umfassten Prozessoreinheit 22 ausgehend von dem beobachteten kinematischen Verhalten des Raupenlaufwerks 16i auf wenigstens einen die Bodenbeschaffenheit charakterisierenden Parameter geschlossen wird.

Letzteres erfolgt, indem das mittels der Erfassungseinrichtung 24 beobachtete kinematische Verhalten einem für das betreffende Raupenlaufwerk 16i vorgegebenen Vorhersagemodell 82 zugeführt wird, anhand dessen wiederum eine Aussage hinsichtlich der Bodenbeschaffenheit getroffen wird.

Das Vorhersagemodell 82, bei dem es sich letztlich um einen Algorithmus handelt, wird vorab aufgrund empirisch durchgeführter Test- und Messreihen eingelernt und in die Prozessoreinheit 22, genauer gesagt in die zugehörige Speichereinheit 28 hochgeladen. Das Vorhersagemodell 82 stellt eine eindeutige Korrelation zwischen dem beobachteten kinematischen Verhalten des Raupenlaufwerks 16i und der jeweiligen Bodenbeschaffenheit her.

Beispielsgemäß ergibt sich das kinematische Verhalten des Raupenlaufwerks 16i aufgrund einer Auslenkung, die fahrtbedingt an einer gegenüber dem Untergrund 46 beweglich gelagerten Fahrgestellkomponente des Raupenlaufwerks 16i, genauer gesagt des Basisteils 56 auftritt, wobei diese vorliegend zu einer mittels des inkrementalen Drehgebers 62 sensorisch erfassten Winkeländerung α um die Drehgelenkachse 60 führt. So bewirkt ein am Laufwerkriemen 38 antriebsbedingt gegenüber dem Untergrund 46 auftretendes Traktionsmoment ein asymmetrisches Einsinken des Raupenlaufwerks 16i entgegen der Fahrtrichtung 84, was sich in einer entsprechenden Winkeländerung α äußert (siehe Fig. 2).

Abweichend davon können bei komplexen Laufwerksanordnungen neben dem Basisteil 56 auch weitere bewegliche Fahrgestellkomponenten vorhanden sein, die der Federung und/oder dem Spannen des Laufwerkriemens 38 mittels zugehöriger Umlenk-, Spann- und Stützrollen 44 dienen. Aus der umfassenden sensorischen Erfassung der insoweit auftretenden Bewegungen ergibt sich ein komplexes und damit besonders unterscheidungskräftiges Auslenkungsmuster, das einen genauen Rückschluss auf die jeweilige Bodenbeschaffenheit zulässt.

Zusätzlich oder alternativ ergibt sich das kinematische Verhalten des Raupenlaufwerks 16i aufgrund einer Normal- und/oder Tangentialbeschleunigung, die fahrtbedingt an einem dem Untergrund 46 zugewandten Abschnitt 86 des Laufwerkriemens 38 auftritt. Hierbei wird die Kenntnis der Normal- und/oder Tangentialbeschleunigung von der Prozessoreinheit 22 zur Ermittlung der Orientierung des betreffenden Riemenabschnitts 86 gegenüber dem Untergrund 46 herangezogen.

Die Normal- und/oder Tangentialbeschleunigung wird mittels des ersten Beschleunigungssensors 40 bestimmt, wobei die Funkschnittstelle 36 einen diese repräsentierenden Datenstrom drahtlos an die zweite Drahtlosschnittstelle 34 und von dort an die Prozessoreinheit 22 übermittelt. Das Fahrzeugchassis 50 dient als Bezugssystem zur Ermittlung der Orientierung des Riemenabschnitts 86, wozu die Prozessoreinheit 22 mittels des zweiten Beschleunigungssensors 48 eine zugehörige Referenz-Orientierung bestimmt.

Da der Laufwerkriemen 38 entlang seines Verlaufs mehrfach umgelenkt wird, ist zur unterbrechungsfreien Erfassung der Normal- und/oder Tangentialbeschleunigung eine den Umlenkstellen 88 entsprechende Anzahl an Beschleunigungsmessmodulen 52 entlang des Laufwerkriemens 38 verteilt angeordnet. Im vorliegenden Fall sind insgesamt drei identische Beschleunigungsmessmodule 52 vorhanden. Entgegen der Darstellung in Fig. 2 sind die Beschleunigungsmessmodule 52 äquidistant entlang des Laufwerkriemens 38 angebracht.

Zur möglichst genauen Ermittlung der Bodenbeschaffenheit ist vorgesehen, dass das beobachtete kinematische Verhalten des Raupenlaufwerks 16i von der Prozessoreinheit 22 hinsichtlich von in Fahrtrichtung 84 liegenden Bodenunebenheiten 90 bereinigt wird. Die Erfassung der Bodenunebenheiten 90 erfolgt mittels der vorausschauenden Sensoreinrichtungen 68.

Typischerweise handelt es sich bei den die Bodenbeschaffenheit charakterisierenden Parametern um Informationen hinsichtlich einer Bodenfeuchtigkeit, einer Bodenstruktur, einer Bodenverdichtung und/oder eines Bodentyps. Im vorliegenden Beispiel ist die Ermittlung der Bodenbeschaffenheit auf eine in Fig. 2 wiedergegebene Auswahl der vorstehend aufgeführten Parameter beschränkt (Bodenstruktur, Bodenverdichtung und Bodentyp), wobei Angaben hinsichtlich eines aktuellen Feuchtegehalts, mithin der Bodenfeuchtigkeit, aus alternativen Datenquellen stammen und so zu einer verbesserten Genauigkeit der verbleibenden Parameter beitragen. Der Feuchtegehalt ergibt sich beispielsweise aus Wetterinformationen, besser noch aus einer vor Ort durchgeführten Bodenmessung. Die Ergebnisse der Bodenmessung werden über die erste Drahtlosschnittstelle 30 in die Speichereinheit 28 hochgeladen, sodass die Prozessoreinheit 22 auf diese Zugriff hat.

Um die im Hinblick auf die Bodenbeschaffenheit gewonnenen Informationen insbesondere beim erneuten Befahren der selben Ackerfläche nutzbar zu machen, werden die ermittelten Parameter durch Verknüpfung mit den seitens des Navigationssystems 64 bereitgestellten Positionsinformationen verortet und als Datensatz über die erste Drahtlosschnittstelle 30 an den zentralen Datenserver 32 übermittelt. Von dort können diese zu Zwecken der Schlagplanerstellung bzw. Prozessoptimierung von einem Anwender heruntergeladen werden.

Da der von dem jeweiligen Raupenlaufwerk 16i verursachte Kompressionseffekt ebenfalls abhängig ist vom Umfang des am Laufwerkriemen 38 gegenüber dem Untergrund 46 antriebsbedingt auftretenden Traktionsmoments und/oder der in dem durch das Drehgelenk 58 gebildeten Anlenkpunkt des Raupenlaufwerks 16i gegenüber dem Fahrzeugchassis 50 gewichtsbedingt wirkenden Achslast, werden von der Prozessoreinheit 22 zur Verbesserung der Genauigkeit des Vorhersagemodells 82 über den CAN-Datenbus 72 weiterhin entsprechende Informationen des Antriebssystems 74 und/oder des Achslastsensors 76 berücksichtigt.

Der Vollständigkeit halber sei angemerkt, dass die Verwendung des erfindungsgemäßen Verfahrens nicht auf landwirtschaftliche Traktoren beschränkt ist, vielmehr lässt sich dieses auch bei beliebigen anderen Arbeitsmaschinen oder Arbeitsgeräten einsetzen, sofern diese mit einem Raupenlaufwerk ausgestattet sind.

## Patentansprüche

1. Verfahren zur Ermittlung einer Bodenbeschaffenheit, bei dem mittels einer Erfassungseinrichtung (24) ein durch Einsinken verursachtes kinematisches Verhalten eines Raupenlaufwerks (16a,...,16d) beim Befahren eines kompressiblen Untergrunds (46) beobachtet wird, wobei von einer Bewertungseinrichtung (26) ausgehend von dem beobachteten kinematischen Verhalten des Raupenlaufwerks (16a,...,16d) auf wenigstens einen die Bodenbeschaffenheit charakterisierenden Parameter geschlossen wird, wobei sich das kinematische Verhalten aufgrund einer fahrtbedingt an einer gegenüber dem Untergrund (46) beweglich gelagerten Fahrgestellkomponente des Raupenlaufwerks (16a,...,16d) auftretenden Auslenkung ergibt,
**dadurch gekennzeichnet, dass** es sich bei der Fahrgestellkomponente um ein über ein Drehgelenk (58) schwenkbar an einem Fahrzeugchassis (50) angebrachtes Basisteil (56) handelt, sodass sich die zu beobachtende Auslenkung aus einer mittels der Erfassungseinrichtung (24) sensorisch erfassbaren Winkeländerung (α) des Basisteils (56) um die Drehgelenkachse (60) ergibt.

2. Verfahren nach Anspruch 1, **dadurch gekennzeichnet, dass** sich das kinematische Verhalten redundant aufgrund einer Normal- und/oder Tangentialbeschleunigung, die fahrtbedingt an einem dem Untergrund (46) zugewandten Abschnitt (86) des Laufwerkriemens (38) auftritt, ergibt.

3. Verfahren nach Anspruch 2, **dadurch gekennzeichnet, dass** die Normal- und/oder Tangentialbeschleunigung mittels eines in den Riemenabschnitt (86) eingebetteten Beschleunigungssensors (40) als Bestandteil der Erfassungseinrichtung (24) bestimmt wird, wobei ein diese repräsentierender Datenstrom drahtlos an die Bewertungseinrichtung (26) übermittelt wird.

4. Verfahren nach Anspruch 3, **dadurch gekennzeichnet, dass** der Laufwerkriemen (38) entlang seines Verlaufs mehrfach umgelenkt wird, wobei eine den Umlenkstellen (88) entsprechende Anzahl an Beschleunigungssensoren (40) entlang des Laufwerkriemens (38) verteilt angeordnet ist.

5. Verfahren nach wenigstens einem der vorhergehenden Ansprüche, **dadurch gekennzeichnet, dass** das beobachtete kinematische Verhalten des Raupenlaufwerks (16a,...,16d) von der Bewertungseinrichtung (26) hinsichtlich von in Fahrtrichtung (84) liegenden Bodenunebenheiten (90) bereinigt wird.

6. Verfahren nach wenigstens einem der vorhergehenden Ansprüche, **dadurch gekennzeichnet, dass** es sich bei den die Bodenbeschaffenheit charakterisierenden Parametern um Informationen hinsichtlich einer Bodenfeuchtigkeit, einer Bodenstruktur, einer Bodenverdichtung und/oder eines Bodentyps handelt.

7. Verfahren nach wenigstens einem der vorhergehenden Ansprüche, **dadurch gekennzeichnet, dass** die ermittelten Parameter mittels eines mit der Bewertungseinrichtung (26) kommunizierenden Navigationssystems (64) durch Verknüpfung mit einer zugehörigen kartografischen Position verortet und als Datensatz an einen zentralen Datenserver (32) übermittelt werden.

## Claims

1. Method for ascertaining a soil condition, in which a kinematic behaviour of a track drive (16a, ..., 16d) caused by sinking when driving on a compressible underlying surface (46) is observed by means of a detection device (24), wherein at least one parameter characterizing the soil condition is concluded by an assessment device (26) starting from the observed kinematic behaviour of the track drive (16a, ..., 16d), wherein the kinematic behaviour results due to a deflection, which occurs because of travel on a chassis component of the track drive (16a, ..., 16d) mounted so it is movable in relation to the underlying surface (46), **characterized in that** the chassis component is a base part (56) attached via a pivot joint (58) in a pivotable manner on a vehicle chassis (50), such that the observed deflection results from a change of angle (α) of the base part (56) about the pivot joint axis (60) as detectable using sensors by means of the detection device (24).

2. Method according to Claim 1, **characterized in that** the kinematic behaviour results redundantly due to a normal and/or tangential acceleration, which occurs because of travel on a section (86) of the drive belt (38) facing towards the underlying surface (46).

3. Method according to Claim 2, **characterized in that** the normal and/or tangential acceleration is determined by means of an acceleration sensor (40) embedded in the belt section (86) as part of the detection device (24), wherein a data stream representing this is transmitted wirelessly to the assessment device (26).

4. Method according to Claim 3, **characterized in that** the drive belt (38) is deflected multiple times along its course, wherein a number of acceleration sensors (40) corresponding to the deflection points (88) is arranged distributed along the drive belt (38).

5. Method according to at least one of the preceding claims, **characterized in that** the observed kinematic behaviour of the track drive (16a,...,16d) is filtered by the assessment device (26) with respect to soil irregularities (90) located in the travel direction (84).

6. Method according to at least one of the preceding claims, **characterized in that** the parameters characterizing the soil condition are items of information with respect to a soil moisture, a soil structure, a soil compaction, and/or a soil type.

7. Method according to at least one of the preceding claims, **characterized in that** the ascertained parameters are located by means of a navigation system (64) communicating with the assessment device (26) by linking to an associated cartographic position and transmitted as a data set to a central data server (32).

## Revendications

1. Procédé de détermination d'un état du sol dans lequel un comportement cinématique provoqué par l'enfoncement d'un mécanisme à chenilles (16a, ..., 16d) lors de son déplacement sur une surface de sol compressible (46) est observé au moyen d'un dispositif de détection (24), un paramètre qui caractérise au moins l'état du sol étant déduit par un dispositif d'évaluation (26) à partir du comportement cinématique observé du mécanisme à chenilles (16a, ..., 16d), le comportement cinématique résultant d'une déviation, qui survient en fonction de la conduite, sur un composant de châssis du mécanisme à chenilles (16a, ..., 16d) monté de manière mobile par rapport à la surface du sol (46), **caractérisé en ce que** le composant de châssis est une partie de base (56) montée pivotante sur un châssis de véhicule (50) par l'intermédiaire d'une articulation tournante (58), de telle sorte que la déviation à observer résulte d'une variation d'angle (α) de la partie de base (56) autour de l'axe d'articulation tournant (60), détectable par des capteurs au moyen du dispositif de détection (24).

2. Procédé selon la revendication 1, **caractérisé en ce que** le comportement cinématique résulte de manière redondante d'une accélération normale et/ou tangentielle qui se produit en raison de la conduite sur une section (86) de la courroie d'entraînement (38) orientée vers la surface du sol (46).

3. Procédé selon la revendication 2, **caractérisé en ce que** l'accélération normale et/ou tangentielle est déterminée au moyen d'un capteur d'accélération (40) intégré dans la section de courroie (86) en tant que partie constitutive du dispositif de détection (24), un flux de données qui représentent celle-ci étant transmis sans fil au dispositif d'évaluation (26).

4. Procédé selon la revendication 3, **caractérisé en ce que** la courroie de roulement (38) est déviée plusieurs fois le long de son parcours, un nombre de capteurs d'accélération (40) qui correspond aux points de renvoi (88) étant disposé de manière répartie le long de la courroie de roulement (38).

5. Procédé selon au moins l'une des revendications précédentes, **caractérisé en ce que** le comportement cinématique observé du mécanisme à chenilles (16a,...,16d) du dispositif d'évaluation (26) est débarrassé des irrégularités du sol (90) se trouvant dans le sens de la marche (84).

6. Procédé selon au moins l'une des revendications précédentes, **caractérisé en ce que** les paramètres caractérisant l'état du sol sont des informations concernant l'humidité du sol, la structure du sol, le compactage du sol et/ou le type de sol.

7. Procédé selon au moins l'une des revendications précédentes, **caractérisé en ce que** les paramètres déterminés sont localisés au moyen d'un système de navigation (64) communiquant avec le dispositif d'évaluation (26), en les associant à une position cartographique correspondante, et sont transmis sous forme de jeu de données à un serveur de données central (32).
